(19) Europäisches Patentamt
European Patent Office
Office européen des brevets

(11) Veröffentlichungsnummer: **0 338 525**
**A1**

(12) **EUROPÄISCHE PATENTANMELDUNG**

(21) Anmeldenummer: **89106994.0**

(51) Int. Cl.[4]: **C07D 285/06 , A01N 43/82**

(22) Anmeldetag: **19.04.89**

(30) Priorität: **22.04.88 DE 3814117**

(43) Veröffentlichungstag der Anmeldung:
**25.10.89 Patentblatt 89/43**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Angermann, Alfred, Dr.**
**Stephanstrasse 23**
**D-1000 Berlin 21(DE)**
Erfinder: **Franke, Helga, Dr.**
**Spiessergasse 6b**
**D-1000 Berlin 27(DE)**
Erfinder: **Rees, Richard, Dr.**
**Speerweg 8**
**D-1000 Berlin 28(DE)**
Erfinder: **Johann, Gerhard, Dr.**
**Hermsdorfer Dann 147**
**D-1000 Berlin 28(DE)**

(54) **Substituierte 2-Thiadiazolylcarbonyl-cyclohexan-1,3-dione und deren Iminoderivate, Verfahren zur Herstellung dieser Verbindungen und ihre Verwendung als Mittel mit herbizider und pflanzenwachstumsregulierender Wirkung.**

(57) Verbindungen der allgemeinen Formel

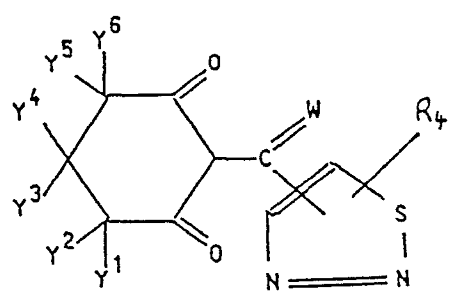

in der W ein Sauerstoffatom oder eine Iminogruppe, und die übrige Symbole verschiedene Substituenten bedeuten, Verfahren zu Herstellung dieser Verbindungen und ihre Verwendung als Mittel mit herbizider und pflanzenwachstungsregulierender Wirkung.

EP 0 338 525 A1

# SUBSTITUIERTE 2-THIADIAZOLYLCARBONYL-CYCLOHEXAN-1,3-DIONE UND DEREN IMINODERIVATE. VERFAHREN ZUR HERSTELLUNG DIESER VERBINDUNGEN UND IHRE VERWENDUNG ALS MITTEL MIT HERBIZIDER UND PFLANZENWACHSTUMSREGULIERENDER WIRKUNG

Die Erfindung betrifft neue substituierte 2-Thiadiazolylcarbonyl-cyclohexan-1,3-dione und deren Iminoderivate, Verfahren zur Herstellung dieser Verbindungen und ihre Verwendung als Mittel mit herbizider und pflanzenwachstumsregulierender Wirkung.

Es ist bereits bekannt, daß gewisse Cyclohexandioncarbonsäurederivate eine herbizide Wirkung besitzen (EP-Anmeldungen 0 137 963, 0 186 118 und 0 186 120). Häufig ist die herbizide Wirkung dieser bekannten Verbindungen aber nicht ausreichend, oder es treten Selektivitätsprobleme in wichtigen landwirtschaftlichen Kulturen auf.

Aufgabe der vorliegenden Erfindung ist daher die Bereitstellung von neuen Verbindungen, die in ihren biologischen Eigenschaften den bisher bekannten Verbindungen überlegen sind.

Diese Aufgabe wird gelöst durch neue substituierte 2-Thiadiazolylcarbonylcyclohexan-1,3-dione und deren Iminoderivate der allgemeinen Formel I

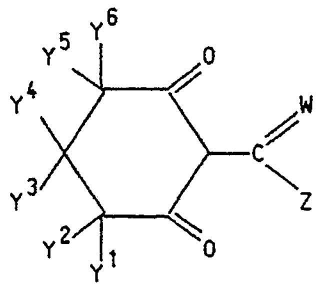

(I) ,

in der

$Y^1$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest, einen 1-($C_1$-$C_4$-Alkoxyimino)-$C_1$-$C_4$-alkylrest, einen 1-($C_3$-$C_4$-Alkenyloxyimino)-$C_1$-$C_4$-alkylrest, einen 1-($C_3$-$C_4$-Alkinyloxyimino)-$C_1$-$C_4$-alkylrest oder eine der Gruppen $-(CH_2)_mCO_2M$, $-(CH_2)_mCO_2R^1$ oder $-(CH_2)_mCONR^2R^3$.

$Y^2$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$ oder

$Y^1$ und $Y^2$ zusammen mit dem benachbarten Kohlenstoffatom einen $C_3$-$C_6$-Cycloalkylrest,

$Y^3$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$,

$Y^4$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$ oder

$Y^3$ und $Y^4$ zusammen mit dem benachbarten Kohlenstoffatom einen $C_3$-$C_6$-Cycloalkylrest oder eine Carbonylgruppe.

$Y^5$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$,

$Y^6$ ein Wasserstoffatom, ein Halogenatom, ein Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$ oder

$Y^5$ und $Y^6$ zusammen mit dem benachbarten Kohlenstoffatomen einen $C_3$-$C_6$-Cycloalkylrest,

W ein Sauerstoffatom oder die Gruppe $=N-R^2$,

Z einen der Thiadiazolylreste

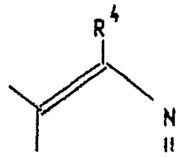

oder

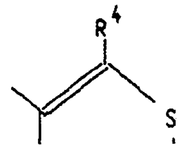

m 0, 1, 2, 3 oder 4,

M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

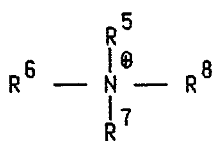

,

$R^1$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,

$R^2$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,

$R^3$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest oder

$R^2$ und $R^3$ gemeinsam mit dem benachbarten Stickstoffatom eine Morpholinogruppe, eine Piperidinogruppe

oder eine Pyrrolidinogruppe,

$R^4$ ein Wasserstoffatom, ein Halogenatom, eine Cyanogruppe, einen $C_1$-$C_{18}$-Alkylrest, einen ein- oder mehrfach, gleich oder verschieden durch Halogen, Cyano oder eine der Gruppen $-OR^1$ oder $-S(O)_nR^1$ substituierten $C_1$-$C_{18}$-Alkylrest, einen $C_2$-$C_{12}$-Alkenylrest, einen ein- oder mehrfach, gleich oder verschieden durch Halogen, Cyano oder eine der Gruppen $-OR^1$ oder $-S(O)_nR^1$ substituierten $C_2$-$C_{12}$-Alkenylrest, einen $C_2$-$C_{12}$-Alkinylrest, einen ein- oder mehrfach, gleich oder verschieden durch Halogen, Cyano oder eine der Gruppen $-OR^1$ oder $-S(O)_nR^1$ substituierten $C_2$-$C_{12}$-Alkinylrest, einen Phenylrest, einen ein- oder mehrfach, gleich oder verschieden durch Halogen, Cyano, $C_1$-$C_4$-Alkyl oder eine der Gruppen $-OR^1$ oder $-S(O)_nR^1$ substituierten Phenylrest, einen Phenyl-$C_1$-$C_2$-alkylrest, einen ein- oder mehrfach, gleich oder verschieden durch Halogen, Cyano, $C_1$-$C_4$-Alkyl oder eine der Gruppen $-OR^1$ oder $S(O)_nR^1$ substituierten Phenyl-$C_1$-$C_2$-alkylrest oder eine der Gruppen $-(CH_2)_mCO_2R^1$, $-COR^1$, $-(CH_2)_mCO_2M$, $-(CH_2)_mCONR^2R^3$, $-NHCONR^2R^3$, $-NHCO_2R^1$, $-NHCOR^1$, $-NHSO_2R^1$, $-(CH_2)_mNR^2R^3$, $-S(O)_nR^1$, $-OR^1$, $-CH=NNHR^1$ oder $-CH=NOR^1$,

$R^5$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest,

$R^6$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest,

$R^7$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest,

$R^8$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehr fach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest, und

n 0, 1 oder 2

bedeuten, und die, falls W für ein Sauerstoffatom steht, daraus abgeleiteten Salze der allgemeinen Formel II

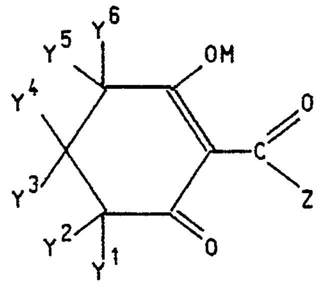

(II) ,

in der M, $Y^1$, $Y^2$, $Y^3$, $Y^4$, $Y^5$, $Y^6$ und Z die unter der allgemeinen Formel I genannten Bedeutungen haben.

Die Bezeichnung "Halogen" umfaßt in diesem Zusammenhang Fluor, Chlor, Brom und Jod. Unter den Begriffen "Alkyl", "Alkenyl" und "Alkinyl" sind sowohl geradkettige als auch verzweigte Kohlenwasserstoffreste zu verstehen.

Die erfindungsgemäßen Verbindungen der allgemeinen Formel I können auch als Tautomere mit den allgemeinen Formeln I′ und I″ vorliegen

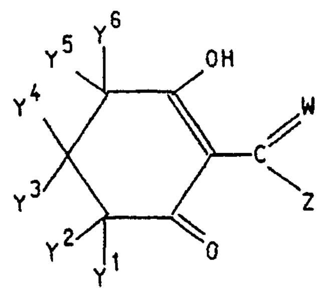

(I′) ,

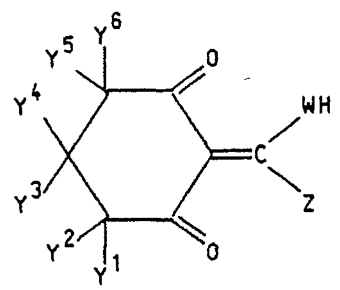

(I′′) ,

in denen W, $Y^1$, $Y^2$, $Y^3$, $Y^4$, $Y^5$, $Y^6$ und Z die unter der allgemeinen Formel I genannten Bedeutungen haben. Diese Strukturen werden ebenfalls durch die vorliegende Erfindung umfaßt, aus Gründen der Einfachheit wird aber jeweils nur die Struktur der allgemeinen Formel I angegeben.

Die erfindungsgemäßen Verbindungen lassen sich herstellen, indem man

A) falls W ein Sauerstoffatom bedeutet, eine Verbindung der allgemeinen Formel III

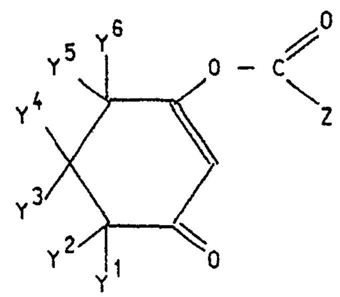

(III) ,

in der $Y^1$, $Y^2$, $Y^3$,$Y^4$, $Y^5$, $Y^6$ und Z die unter der allgemeinen Formel I genannten Bedeutungen haben, einer katalytisch geförderten Umlagerungsreaktion unterwirft oder

B) falls W die Gruppe $=N\text{-}R^2$ bedeutet, eine Verbindung der allgemeinen Formel IV

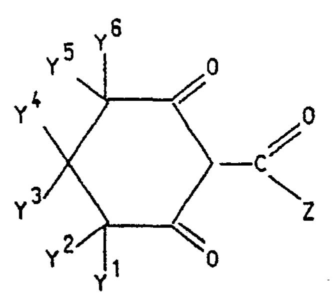

(IV) ,

in der $Y^1$, $Y^2$, $Y^3$, $Y^4$, $Y^5$, $Y^6$ und Z die unter der allgemeinen Formel I genannten Bedeutungen haben, mit Aminen der allgemeinen Formel V

$H_2N - R^2$ (V),

in der $R^2$ die unter der allgemeinen Formel I genannte Bedeutung hat, kondensiert.

Als Katalysatoren für die Umlagerungsreaktion A) eignen sich Pyridinderivate, wie zum Beispiel 4-(N,N-Dialkylamino)-pyridine, vorzugsweise 4-(N,N-Dimethylamino)-pyridin, N-Alkylimidazole, wie zum Beispiel N-Methylimidazol, Metallcyanide, wie zum Beispiel Kupfer-I-cyanid, Natriumcyanid, Kaliumcyanid und Zinkcyanid, 2-Hydroxyisobutansäurenitril, oder Lewis-Säuren, wie zum Beispiel Zinkchlorid oder Aluminiumchlorid.

Die Umsetzung wird unter Feuchtigkeitsausschluß und vorzugsweise in Anwesenheit eines inerten Lösungsmittels durchgeführt. Als besonders geeignet haben sich hierfür aromatische Kohlenwasserstoffe, wie Benzol, Toluol und Xylol, Halogenkohlenwasserstoffe, wie zum Beispiel Tetrachlorkohlenstoff, Trichlormethan und Dichlormethan, Acetonitril oder organische Basen, wie Triethylamin und Pyridin, erwiesen.

Die Ausgangsstoffe der allgemeinen Formel III erhält man durch Acylierung eines β-Dioxocyclohexanderivates der allgemeinen Formel VI

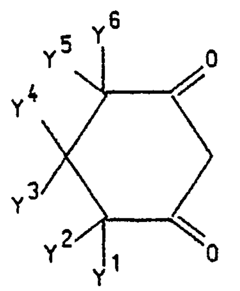

(VI) ,

in der $Y^1$, $Y^2$, $Y^3$, $Y^4$, $Y^5$, $Y^6$ die unter der allgemeinen Formel I genannten Bedeutungen haben, mit Verbindungen der allgemeinen Formel VII

$X - \overset{\overset{\displaystyle O}{\|}}{C} - Z$ (VII) ,

in der Z die unter der allgemeinen Formel I angegebene Bedeutung hat und X für eine Fluchtgruppe, wie zum Beispiel Halogen, vorzugsweise für ein Chlor- oder Bromatom, steht.

Diese Ayclierungsreaktion wird gegebenenfalls in Anwesenheit eines inerten Lösungsmittels und eines säurebindenden Mittels durchgeführt. Als solche säurebindenden Mittel kommen verschiedene organische und anorganische Basen in Betracht, als besonders geeignet haben sich jedoch Triethylamin und Pyridin erwiesen.

Das Herstellungsverfahren B) wird zweckmäßigerweise unter wasserabspaltenden Bedingungen und gegebenenfalls in Anwesenheit eines geeigneten Lösungsbeziehungsweise Verdünnungsmittels durchgeführt. Als besonders geeignet haben sich hierfür chlorierte Kohlenwasserstoffe, wie Dichlormethan oder Chloroform, und Alkohole, wie Methanol, Ethanol und 2-Propanol, erwiesen.

Die Wasserabspaltung kann gegebenenfalls durch die üblichen Methoden unterstützt werden, beispielsweise durch Zugabe eines wasserbindenden Mittels wie Molekularsieb (3Å) oder Magnesiumsulfat. Auch die Entfernung des gebildeten Wassers durch Destillation beziehungsweise azeotrope Destillation kann von Vorteil sein.

Bei den Herstellungsmethoden A) und B) können - neben den bereits erwähnten - auch jeweils alle solchen Lösungs- beziehungsweise Verdünnungsmittel zum Einsatz kommen, die gegenüber den jeweiligen Reaktanden inert sind. Beispiele für solche Lösungsmittel beziehungsweise Verdünnungsmittel sind aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid, Trichlorethylen und Chlorbenzol, Ether, wie zum Beispiel Diethylether, Methylethylether, Methyl-t-butylether, Diisopropylether, Dibutylether, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide, wie zum Beispiel Dimethylsulfoxid, und Sulfone, wie zum Beispiel Sulfolan, sowie Basen, wie zum Beispiel Pyridin.

Alle Reaktionen werden vorzugsweise unter dem Druck der Umgebung durchgeführt, wenngleich sie auch bei erhöhtem oder vermindertem Druck ausgeführt werden könnten. Sie können ferner innerhalb eines breiten Temperaturbereichs durchgeführt werden. Im allgemeinen werden sie bei einer Temperatur zwischen -20 °C und dem Siedepunkt des Reaktionsgemisches, vorzugsweise bei 0 °C bis 150 °C, durchgeführt.

Die Gegenwart eines zusätzlichen Reaktionskatalysators kann von Vorteil sein. Als solche Katalysatoren sind Kaliumjodid und Oniumverbindungen, wie quarternäre Ammonium-, Phosphonium-, Arsonium- und Sulfoniumverbindungen, geeignet. Ebenfalls geeignet sind Polyglycolether, insbesondere cyclische, wie zum Beispiel 18-Krone-6, und tertiäre Amine, wie zum Beispiel Tributylamin. Bevorzugt sind quarternäre Ammoniumverbindungen, wie zum Beispiel Benzyltriethylammoniumbromid und Tetrabutylammoniumbromid.

Die nach den oben genannten Verfahren hergestellten erfindungsgemäßen Verbindungen können nach den üblichen Methoden aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion. Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb- und geruchlose Flüssigkeiten oder Kristalle dar, die bedingt löslich in Wasser, aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, Sulfoxiden, wie Dimethylsulfoxid, und - aufgrund ihres in der Regel sauren Charakters - in Basen wie Alkalihydroxid -, Alkalicarbonat- und Alkalihydrogencarbonatlösungen, Triethylamin oder Pyridin sind.

Die bei dem Herstellungsverfahrens A) zur Acylierung benötigten Verbindungen der allgemeinen Formel VII sind entweder literaturbekannt oder können in Anlehnung an literaturbekannte Methoden hergestellt werden. Einschlägige Techniken sind beispielsweise in J. Am. Chem. Soc. 77, 5359 (1955) oder Chem. Ber. 99, 1618 (1966) beschrieben.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,03 bis 5 kg/ha.

Die erfindungsgemäßen Wirkstoffe können auch als Defoliant, Desiccant und als Krautabtötungsmittel verwendet werden.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 36, No. 12, 1987, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Die erfindungsgemäßen Verbindungen verursachen darüberhinaus sowohl qualitative wie quantitative Veränderungen von Pflanzen als auch Veränderungen im Metabolismus der Pflanzen und sind daher in die Klasse der Pflanzenwachstumsregulatoren einzustufen, die sich durch folgende Anwendungsmöglichkeiten

auszeichnen:
- Hemmung des vegetativen Wachstums bei holzigen und krautigen Pflanzen zum Beispiel an Straßenrändern, Gleisanlagen und anderen, um ein zu üppiges Wachstum zu unterbinden. Wuchshemmung beim Getreide, um das Lagern oder Umknicken zu unterbinden, bei Baumwolle zur Ertragserhöhung.
- Beeinflussung dr Verzweigung von vegetativen und generativen Organen bei Zier- und Kulturpflanzen zur Vermehrung des Blütenansatzes oder bei Tabak und Tomate zur Hemmung von Seitentrieben.
- Verbesserung der Fruchtqualität, zum Beispiel eine Zuckergehaltssteigerung beim Zuckerrohr, bei Zuckerrüben oder bei Obst und eine gleichmäßigere Reife des Erntegutes, die zu höheren Erträgen führt.
- Erhöhung der Widerstandskraft gegen Streß, so zum Beispiel gegen klima tische Einflüsse, wie kälte und Trockenheit, aber auch gegen phytotoxische Einflüsse von Chemikalien.
- Beeinflussung des Latexflusses bei Gummipflanzen.
- Ausbildung parthenokarper Früchte, Pollensterilität und Geschlechtsbe einflussung sind ebenfalls Anwendungsmöglichkeiten.
- Kontrolle der Keimung von Samen oder des Austriebs von Knospen.
- Entlaubung oder Beeinflussung des Fruchtfalles zur Ernteerleichterung.

Die erfindungsgemäßen Verbindungen eignen sich insbesondere zur Hemmung des vegetativen Wachstums bei verschiedenen Pflanzenarten. Eine solche Wuchshemmung wird bisher in der Praxis mit Substanzen verursacht, die entweder nicht konsistent wirken oder in ihrer Wirkung schächer sind als die erfindungsgemäßen Verbindungen.

Dabei handelt es sich zum Beispiel um Stoffe für verschiedene Getreidearten, die eingesetzt werden, um eine Halmverkürzung zu erreichen und der Pflanze eine größere Standfestigkeit zu verleihen. Darüber hinaus können mit einer derartigen Wirkung Pflanzen im Wuchs gehemmt werden, deren Wachstum an bestimmten Standorten nicht erwünscht ist, so zum Beispiel an Straßenrändern, an Gleisanlagen oder auf Flugplätzen. Auch Grasarten im Rasen können beeinflußt werden, so daß seltener geschnitten werden muß. Wuchshemmende Effekte werden mit solchen Substanzen im allgemeinen auch bei Kulturpflanzen erzielt, die sich durch den Austrieb ruhender Knospen zu intensiv verzweigen, wie zum Beispiel Tabak.

Bei Baumwolle können Wuchshemmungen zu einem gleichmäßigen Ausreifen der Kapseln und dadurch zu höheren Erträgen führen.

Bei Obstbäumen führt die gezielte Beeinflussung des vegetativen Wachstums zu Ertragssteigerungen und arbeitswirtschaftlichen Vorteilen.

Bei Zierpflanzen erreicht man durch eine Wuchsreduktion oft, daß mehr Pflanzen pro Stellfläche angezogen werden können.

Die erfindungsgemäßen Verbindungen entfalten ihre Wirkung sowohl bei Vor-als auch bei Nachauflaufbehandlung. Die Aufwandmengen betragen je nach Anwendungsziel 0,001 bis 5 kg/ha, gegebenenfalls können auch höhere Aufwandmengen eingesetzt werden.

Die Anwendungszeit richtet sich nach dem Anwendungziel und den klimatischen Bedingungen.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 1 bis 90 Gewichtsprozent Wirkstoff, etwa 99 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

| A) **Spritzpulver** | | |
|---|---|---|
| 1.) | 20 | Gewichtsprozent Wirkstoff |
| | 68 | Gewichtsprozent Kaolin |
| | 10 | Gewichtsprozent Calciumsalz der Ligninsulfonsäure |
| | 2 | Gewichtsprozent Dialkylnaphthalinsulfonat |
| 2.) | 40 | Gewichtsprozent Wirkstoff |
| | 25 | Gewichtsprozent Kaolin |
| | 25 | Gewichtsprozent kolloidale Kieselsäure |
| | 8 | Gewichtsprozent Calciumsalz der Ligninsulfonsäure |
| | 2 | Gewichtsprozent Natriumsalz des N-Methyl-N-oleyl-taurins |
| b) **Emulsionskonzentrat** | | |
| | 20 | Gewichtsprozent Wirkstoff |
| | 75 | Gewichtsprozent Isophoron |
| | 2 | Gewichtsprozent ethoxyliertes Rizinusöl |
| | 3 | Gewichtsprozent Calciumsalz der Dodecylphenylsulfonsäure |
| C) **Granulat** | | |
| | 1,66 | Gewichtsprozent Wirkstoff |
| | 98,34 | Gewichtsprozent Attapulgitgranulat |

Die folgenden Beispiele erläutern die Herstellung der erfindungsgemäßen Verbindungen:

**Beispiel 1.01**

2-(4-Methyl-1,2,3-thiadiazol-5-ylcarbonyl)-cyclohexan-1,3-dion

Zu 10,00 g (89,2 mmol) Cyclohexan-1,3-dion, gelöst in 175 ml Dichlormethan und 20 ml Triethylamin werden bei Eistemperatur langsam 16,00 g (98,4 mmol) 4-Methyl-1,2,3-thiadiazol-5-carbonsäurechlorid getropft und anschließend noch 3 Stunden bei Raumtemperatur nachgerührt. Man gießt auf Eis, trennt ab und wäscht die organische Phase mit 1 N Salzsäure, gesättigter Bicarbonatlösung und Wasser, trocknet (Magnesiumsulfat) und engt ein. Das so erhaltene 3-(4-Methyl-1,2,3-thiadiazol-5-ylcarbonyloxy)-2-cyclohexen-1-on wird in 120 ml Acetonitril, 25 ml Triethylamin und 3 ml Acetoncyanhydrin 24 Stunden bei Raumtemperatur gerührt. Der Ansatz wird in 100 ml Diethylether aufgenommen, mit 100 ml 4 N Salzsäure gewaschen und zweimal mit je 50 ml 5%iger Kaliumcarbonatlösung extrahiert. Nach Überschichten dieser alkalischen Phase mit 100 ml Diethylether säuert man mit 4 N Salzsäure, bis pH 3 an, trennt ab, wäscht mit Wasser, trocknet (Magnesiumsulfat) und rotiert ein. Umkristallisation aus Essigester liefert die gewünschte Substanz in Form farbloser Nadeln.
Ausbeute: 15,30 g = 72 % der Theorie
Fp.: 48-49 °C

Analog wurden die folgenden erfindungsgemäßen Verbindungen hergestellt:

| Beispiel | Name der Verbindung | Physikalische Konstante |
|---|---|---|
| 1.02 | 5,5-Dimethyl-2-(4-methyl-1,2,3-thiadiazol-5-ylcarbonyl)-cyclohexan-1,3-dion | Fp.: 68-70°C |
| 1.03 | 3,5-Dioxo-4-(4-methyl-1,2,3-thiadiazol-5-ylcarbonyl)-cyclohexancarbonsäureethylester | Fp.: 58-60°C |
| 1.04 | 5-Methyl-2-(4-methyl-1,2,3-thiadiazol-5-ylcarbonyl)-cyclohexan-1,3-dion | Fp.: 59-60°C |
| 1.05 | 4,4-Dimethyl-2-(4-methyl-1,2,3-thiadiazol-5-ylcarbonyl)-cyclohexan-1,3-dion | Fp.: 57°C |
| 1.06 | 2-(1,2,3-Thiadiazol-5-ylcarbonyl)-cyclohexan-1,3-dion | Fp.: 145-147°C |
| 1.07 | 5,5-Dimethyl-2-(1,2,3-thiadiazol-5-ylcarbonyl)-cyclohexan-1,3-dion | Fp.: 109°C |
| 1.08 | 4,4-Dimethyl-2-(1,2,3-thiadiazol-5-ylcarbonyl)-cyclohexan-1,3-dion | Fp.: 82°C |
| 1.09 | 3,5-Dioxo-4-(1,2,3-thiadiazol-5-ylcarbonyl)-cyclohexancarbonsäure-ethylester | Fp.: 90°C |
| 1.10 | 5-Isopropyl-2-(4-methyl-1,2,3-thiadiazol-5-ylcarbonyl)-cyclohexan-1,3-dion | $n_D^{20}$ : 1,5812 |

| Beispiel | Name der Verbindung | Physikalische Konstante |
| --- | --- | --- |
| 1.11 | 3,5-Dioxo-1-methyl-4-(4-methyl-1,2,3-thiadiazol-5-ylcarbonyl)-cyclohexancarbonsäureethylester | $n_D^{20}$ : 1,5615 |
| 1.12 | 2-(1,2,3-Thiadiazol-4-ylcarbonyl)-cyclohexan-1,3-dion | Fp.: 93-95°C |
| 1.13 | 2-(4-Methoxycarbonyl-1,2,3-thiadiazol-5-ylcarbonyl)-cyclohexan-1,3-dion | Fp.: 78-80°C |
| 1.14 | 2-(4-Ethyl-1,2,3-thiadiazol-5-ylcarbonyl)-cyclohexan-1,3-dion | $n_D^{20}$ : 1,5887 |
| 1.15 | 2-(4-Methoxymethyl-1,2,3-thiadiazol-5-ylcarbonyl)-cyclohexan-1,3-dion | $n_D^{20}$ : 1,5944 |
| 1.16 | 4,4,6,6-Tetramethyl-2-(4-methyl-1,2,3-thiadiazol-5-ylcarbonyl)-cyclohexan-1,3,5-trion | Fp.: 70-71°C |
| 1.17 | 2-(4-Ethyl-1,2,3-thiadiazol-5-ylcarbonyl)-4,4,6,6-tetramethyl-cyclohexan-1,3,5-trion | Fp.: 37°C |
| 1.18 | 4,4,6,6-Tetramethyl-2-(4-methoxymethyl-1,2,3-thiadiazol-5-ylcarbonyl)-cyclohexan-1,3,5-trion | $n_D^{20}$ : 1,5668 |
| 1.19 | 4-(1-Ethoxyiminoethyl)-2-(4-methyl-1,2,3-thiadiazol-5-ylcarbonyl)-cyclohexan-1,3-dion | $n_D^{20}$ : 1,5848 |

**Beispiel 2.01**

3,5-Dioxo-4-(4-methyl-1,2,3-thiadiazol-5-ylcarbonyl)-cyclohexancarbonsäure

4,50 g (14,5 mmol) 3,5-Dioxo-4-(4-methyl-1,2,3-thiadiazol-5-ylcarbonyl)-cyclohexancarbonsäureethylester werden in 30ml 5 %iger Natriumhydroxid-Lösung 30 Minuten auf dem Wasserbad erhitzt, anschließend mit Ether überschichtet und mit Salzsäure auf pH 3 eingestellt. Abtrennen und Einrotieren der wäßrigen Phase ergibt nach Umkristallisation aus Methanol das Produkt in Form farbloser Kristalle.
Ausbeute: 3,20 g = 78 % der Theorie
Fp.: 185 °C (Zers.)

**Beispiel 2.02**

3,5-Dioxo-4-(1,2,3-thiadiazol-5-ylcarbonyl)-cyclohexancarbonsäureethylester-Natriumsalz

Zu 2,50 g (8,4 mmol) 3,5-Dioxo-4-(1,2,3-thiadiazol-5-ylcarbonyl)-cyclohexancarbonsäureethylester (Beispiele 1.09) in 30 ml Ethanol wird bei Eistemperator die äquivalente Menge ethanolischer Natriumhydroxyd-Lösung getropft und anschließend noch 2 Stunden bei Raumtemperatur nachgerührt. Entfernen des Lösungsmittels im Vakuum ergibt die gewünschte Substanz.
Ausbeute: 2,7 g = 100 % der Theorie
Fp.: 111 °C

Analog wurden die folgenden erfindungsgemäßen Verbindungen hergestellt:

| Beispiel | Name der Verbindung | Physikalische Konstante |
|---|---|---|
| 2.03 | 2-(4-Methyl-1,2,3-thiadiazol-5-yl-carbonyl)-cyclohexan-1,3-dion-Natriumsalz | Fp.: 267° C |
| 2.04 | 2-(4-Methyl-1,2,3-thiadiazol-5-yl-carbonyl)-cyclohexan-1,3-dion-Triethylammoniumsalz | Fp.: 57° C |
| 2.05 | 2-(4-Methyl-1,2,3-thiadiazol-5-yl-carbonyl)-cyclohexan-1,3-dion-Magnesiumsalz | Fp.: > 270° C |
| 2.06 | 2-(4-Methyl-1,2,3-thiadiazol-5-yl-carbonyl)-cyclohexan-1,3-dion-Calciumsalz | Fp.: > 270° C |
| 2.07 | 4,4,6,6-Tetramethyl-2-(4-methyl-1,2,3-thiadiazol-5-ylcarbonyl)-cyclohexan-1,3,5-trion-Magnesiumsalz | Fp.: > 270° C |
| 2.08 | 4,4,6,6-Tetramethyl-2-(4-methyl-1,2,3-thiadiazol-5-ylcarbonyl)-cyclohexan-1,3,5-trion-Calciumsalz | Fp.: > 270° C |

**Beispiel 3.01**

2-[1-(4-Methyl-1,2,3-thiadiazol-5-yl)-1-(phenylimino)-methyl]-cyclohexan-1,3-dion

3,00 g (12,6 mmol) 2-(4-Methyl-1,2,3-thiadiazol-5-ylcarbonyl)-cyclohexan-1,3-dion und 1,5 ml Anilin werden in 30 ml absolutem Ethanol 12 Stunden bei Raumtemperatur gerührt. Der nach Abdestillieren des Lösungsmittels erhaltene Festkörper wird mit Hexan gewaschen und aus Ethanol umkristallisiert.
Ausbeute: 2,90 g = 73,5 % der Theorie
Fp.: 117 °C

Analog wurden die folgenden erfindungsgemäßen Verbindungen hergestellt:

| Beispiel | Name der Verbindung | Physikalische Konstante |
| --- | --- | --- |
| 3.02 | 2-[1-(α-Butylimino)-1-(4-methyl-1,2,3-thiadiazol-5-yl)methyl]-cyclohexan-1,3-dion | Fp.: 55-58° C |
| 3.03 | 5,5-Dimethyl-2-[1-(4-methyl-1,2,3-thiadiazol-5-yl)-1-(phenylimino)-methyl]-cyclohexan-1,3-dion | Fp.: 136° C |
| 3.04 | 2-[1-(α-Butylimino)-1-(4-methyl-1,2,3-thiadiazol-5-yl)methyl]-5,5-dimethylcyclohexan-1,3-dion | Fp.: 82-83° C |
| 3.05 | 3,5-Dioxo-4-[1-(4-methyl-1,2,3-thiadiazol-5-yl)-1-(phenylimino)-methyl]-cyclohexancarbonsäureethylester | Fp.: 90° C |
| 3.06 | 3,5-Dioxo-4-[1-phenylimino-1-(1,2,3-thiadiazol-5-yl)-methyl]-cyclohexancarbonsäureethylester | Fp.: 115-117° C |
| 3.07 | 2-[1-(2-Hydroxyethylimino)-1-(4-methyl-1,2,3-thiadiazol-5-yl)-methyl]-cyclohexan-1,3-dion | Fp.: 124° C |

Die folgenden Beispiele erläutern die Anwendungsmöglichkeiten der erfindungsgemäßen Verbindungen:

**Beispiel A**

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsionen mit 500 Litern Wasser/ha gleichmäßig über die Pflanzen versprüht. Hier zeigt zwei Wochen nach der Behandlung die erfindungsgemäße Verbindung eine hohe Kulturpflanzen-Selektivität in Weizen und Mais bei ausgezeichneter Wirkung gegen das Unkraut. Das Vergleichsmittel zeigte nicht die gleichhohe Wirksamkeit.
In der folgenden Tabelle bedeuten:

0 = nicht geschädigt
4 = total vernichtet

TRZAX = Triticium aestivum
ZEAMX = Zea mays
ABUTH = Abutilon theophrasti
MATCH = Matricaria chamomilla
SEBEX = Sesbania exaltata
SOLSS = Solanum sp.
VERPE = Veronica persica
SETVI = Setaria viridis

| Erfindungsgemäße Verbindung | TRZAX | ZEAMX | ABUTH | MATCH | SEBEX | SOLSS | VERPE | SETVI |
|---|---|---|---|---|---|---|---|---|
| Beispiel 1.01 | 0 | 0 | 4 | 3 | 4 | 4 | 4 | 3 |
| Unbehandelt | 0 | 0 | 0 | 0 | 0 | 0 | 0 | 0 |
| Vergleichsmittel<br>Verbindung 2<br>(aus EP-Anmeldung 0 137 963) | 0 | 1 | 4 | 1 | 2 | 4 | 0 | 2 |

**Beispiel B**

Im Gewächshaus wurden die in der Tabelle aufgeführten Verbindungen mit den ebenfalls erwähnten Aufwandmengen appliziert. Hierzu wurden die Wirkstoffe als Emulsionskonzentrate in Gefäße mit 1500 ml Wasser gegeben. Als Testpflanzenarten wurden Oryza sativa (ORYSA), Cyperus spp. (CYPSS), Sagittaria spp. (SAGSS), Scirpus juncoides (SCPJU) und Eleocharis kuroguwai (ELOKU) im 2-bis 5-Blatt-Stadium eingesetzt. Drei Wochen nach der Applikation wurde die Schädigung der Pflanzen bonitiert, und zwar nach folgendem Schema:

0 = keine Schädigung
1 = schwache Schädigung
2 = mittlere Schädigung
3 = starke Schädigung
4 = total vernichtet
\- = nicht geprüft

| Erfindungsgemäße Verbindungen | Wasserapplikation ppm | ORYSA | CYPSS | SAGSS | SCPJU | ELOKU |
|---|---|---|---|---|---|---|
| Beispiel 1.01 | 30 | 0 | 4 | 4 | 4 | 4 |
| Beispiel 1.06 | 30 | 0 | 4 | - | - | - |
| Beispiel 1.15 | 10 | 0 | 3 | - | 3 | - |
| Beispiel 2.04 | 10 | 0 | 3 | - | - | - |
| Beispiel 3.01 | 10 | 0 | 4 | - | 3 | - |
| Beispiel 3.07 | 10 | 0 | 4 | - | 4 | - |

Wie die Tabelle zeigt, sind die erfindungsgemäßen Verbindungen stark wirksam gegen wichtige Reisunkräuter und gleichzeitig selektiv zu Wasserreis.

**Beispiel C**

Im Gewächshaus wurde die in der Tabelle aufgeführte erfindungsgemäße Verbindung mit der ebenfalls erwähnten Aufwandmenge appliziert. Hierzu wurde der Wirkstoff als Granulat auf die Bodenoberfläche gestreut. Als Testpflanzen wurden Oryza sativa (ORYSA), Cyperus difformis (CYPDI), Scirpus juncoides (SCPJU), Eleocharis acicularis (ELOAC), Monochoria vaginalis (MOOVA) und Eleocharis kuroguwai (ELOKU) im 1- bis 2-Blatt-Stadium eingesetzt. Drei Wochen nach der Applikation wurde die Schädigung der Pflanzen bonitiert, und zwar nach folgendem Schema:

0 = keine Schädigung

1 = schwache Schädigung

2 = mittlere Schädigung

3 = starke Schädigung

4 = total vernichtet

| Erfindungsgemäße Verbindung | kg Wirkstoff/ha | ORYSA | CYPDI | SCPJU | ELOAC | MOOVA | ELOKU |
|---|---|---|---|---|---|---|---|
| Beispiel 1.01 | 1,1 | 1 | 4 | 4 | 4 | 3 | 4 |
| Vergleichsmittel | | | | | | | |
| Pyrazolate | 2,0 | 1 | 4 | 2 | 3 | 2 | 0 |

Wie die Tabelle zeigt, ist die erfindungsgemäße Verbindung stark wirksam gegen wichtige Reisunkräuter und gleichzeitig selektiv zu Wasserreis.

## Beispiel D

Im Gewächshaus wurden die aufgeführten Pflanzenspezies vor dem Auflaufen mit den aufgeführten Verbindungen in einer Aufwandmenge von 1,0 kg Wirkstoff/ha behandelt. Die Verbindungen wurden zu diesem Zweck als Emulsionen mit 500 Litern Wasser/ha gleichmäßig über die Pflanzen versprüht. Hier zeigen drei Wochen nach der Behandlung die erfindungsgemäßen Verbindungen eine hohe Kulturpflanzenselektivität in Weizen und Mais bei ausgezeichneter Wirkung gegen das Unkraut. Das Vergleichsmittel zeigte nicht die gleichhohe Selektivität.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung

TRZAX = Triticum aestivum
ZEAMX = Zea mays
BEAVX = Beta vulgaris altissima
BRSSS = Brassica sp.
HELAN = Helianthus annuus
SETVI = Setaria viridis
PANSS = Panicum maximum
CYPES = Cyperus esculentus
ABUTH = Abutilon theophrasti
GALAP = Galium aparine
MATCH = Matricaria chamomilla
POLSS = Polygonum sp.

| Erfindungsgemäße Verbindung | TRZAX | ZEAMX | BEAVX | BRSSS | HELAN | SETVI | PANSS | CYPES | ABUTH | GALAP | MATCH | POLSS |
|---|---|---|---|---|---|---|---|---|---|---|---|---|
| Beispiel 1.16 | 1 | 0 | 4 | 4 | 3 | 4 | 4 | 4 | 4 | 3 | 4 | 3 |
| Unbehandelt | 0 | 0 | 0 | 0 | 0 | 0 | 0 | 0 | 0 | 0 | 0 | 0 |
| Vergleichsmittel | | | | | | | | | | | | |
| Norflurazon | 3 | 3 | 4 | 4 | 3 | 4 | 4 | 3 | 4 | 3 | 4 | 1 |

**Beispiel E**

Im Gewächshaus wurden die aufgeführten Pflanzenspezies vor dem Auflaufen mit den aufgeführten Verbindungen in einer Aufwandmenge von 3,0 kg Wirkstoff/ha behandelt. Die Verbindungen wurden zu diesem Zweck als Emulsionen mit 500 Litern Wasser/ha gleichmäßig über die Pflanzen versprüht. Hier

zeigen drei Wochen nach der Behandlung die erfindungsgemäßen Verbindungen eine ausgezeichnete Wirkung gegen das Unkraut.

In der folgenden Tabelle bedeuten:

0 = keine Schädigung

1 = 1 - 24 % Schädigung

2 = 25 - 74 % Schädigung

3 = 75 - 89 % Schädigung

4 = 90 - 100 % Schädigung

AVEFA = Avena fatua

SETVI = Setaria viridis

ABUTH = Abutilon theophrasti

GALAP = Galium aparine

MATCH = Matricaria chamomilla

| Erfindungsgemäße Verbindungen | AVEFA | SETVI | ABUTH | GALAP | MATCH |
|---|---|---|---|---|---|
| Beispiel 1.04 | 3 | 4 | 4 | 3 | 4 |
| Beispiel 1.05 | 3 | 4 | 4 | 4 | 4 |
| Beispiel 1.06 | 0 | 4 | 2 | 3 | 3 |
| Beispiel 1.07 | 0 | 1 | 0 | 4 | 2 |
| Beispiel 1.14 | 0 | 2 | 4 | 3 | 3 |
| Beispiel 1.15 | 0 | 3 | 4 | 4 | 4 |
| Beispiel 1.17 | 2 | 2 | 4 | 4 | 4 |
| Beispiel 1.19 | 0 | 0 | 3 | 3 | 4 |
| Beispiel 2.03 | 2 | 4 | 4 | 1 | 2 |
| Beispiel 2.04 | 0 | 4 | 4 | 4 | 4 |
| Beispiel 3.01 | 3 | 4 | 4 | 3 | 4 |
| Beispiel 3.02 | 3 | 4 | 4 | 4 | 4 |
| Beispiel 3.03 | 0 | 3 | 1 | 4 | 2 |
| Beispiel 3.04 | 0 | 2 | 0 | 2 | 3 |
| Beispiel 3.07 | 0 | 4 | 4 | - | 4 |

**Beispiel F**

Im Gewächshaus wurden die aufgeführten Pflanzenspezies vor dem Auflaufen mit den aufgeführten Verbindungen in einer Aufwandmenge von 3,0 kg Wirkstoff/ha behandelt. Die Verbindungen wurden zu diesem Zweck als Emulsionen mit 500 Litern Wasser/ha gleichmäßig über die Pflanzen versprüht. Hier zeigen zwei Wochen nach der Behandlung die erfindungsgemäßen Verbindungen eine ausgezeichnete Wirkung gegen das Unkraut.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung

AVEFA = Avena fatua
SETVI = Setaria viridis
ABUTH = Abutilon theophrasti
GALAP = Galium aparine
MATCH = Matricaria chamomilla

| Erfindungsgemäße Verbindungen | AVEFA | SETVI | ABUTH | GALAP | MATCH |
|---|---|---|---|---|---|
| Beispiel 1.04 | 3 | 3 | 4 | 3 | 2 |
| Beispiel 1.05 | 2 | 2 | 4 | 3 | 4 |
| Beispiel 1.06 | 4 | 3 | 3 | 4 | 4 |
| Beispiel 1.08 | 3 | 2 | 0 | 3 | 0 |
| Beispiel 1.14 | 0 | 3 | 4 | 1 | 3 |
| Beispiel 1.15 | 1 | 4 | 2 | 2 | 2 |
| Beispiel 2.03 | 0 | 3 | 4 | 3 | 4 |
| Beispiel 2.04 | 1 | 2 | 4 | 3 | 3 |
| Beispiel 3.01 | 3 | 4 | 4 | 3 | 4 |
| Beispiel 3.02 | 2 | 4 | 4 | 4 | 4 |
| Beispiel 3.04 | 0 | 1 | 0 | 3 | 0 |
| Beispiel 3.07 | 2 | 0 | 4 | 3 | 4 |

## Ansprüche

1. Substituierte 2-Thiadiazolylcarbonyl-cyclohexan-1,3-dione und deren Iminoderivate der allgemeinen Formel I

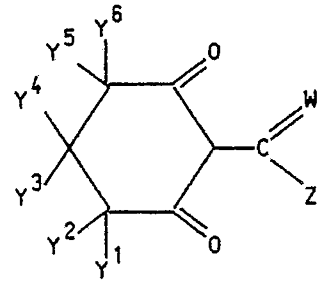

(I) ,

in der

$Y^1$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest, einen 1-($C_1$-$C_4$-Alkoxyimino)-$C_1$-$C_4$-alkylrest, einen 1-($C_3$-$C_4$-Alkenyloxyimino)-$C_1$-$C_4$-alkylrest, einen 1-($C_3$-$C_4$-Alkinyloxyimino)-$C_1$-$C_4$-alkylrest oder eine der Gruppen $-(CH_2)_mCO_2M$, $-(CH_2)_mCO_2R^1$ oder $-(CH_2)_mCONR^2R^3$.

$Y^2$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$ oder

$Y^1$ und $Y^2$ zusammen mit dem benachbarten Kohlenstoffatom einen $C_3$-$C_6$-Cycloalkylrest,

$Y^3$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$- $C_6$-Cycloalkylrest oder eine der Gruppen $-(CH_2)_mCO_2M$, $-(CH_2)_mCO_2R^1$ oder $-(CH_2)_mCONR^2R^3$,

$Y^4$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$ oder

$Y^3$ und $Y^4$ zusammen mit dem benachbarten Kohlenstoffatom einen $C_3$-$C_6$-Cycloalkylrest oder eine Carbonylgruppe.

$Y^5$ ein Wasserstoffatom, ein Halogenatom, eine Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$,

$Y^6$ ein Wasserstoffatom, ein Halogenatom, ein Cyanogruppe, einen $C_1$-$C_{18}$-Alkylrest, einen durch Halogen oder Cyano substituierten $C_1$-$C_{18}$-Alkylrest, einen $C_2$-$C_{12}$-Alkenylrest, einen durch Halogen oder Cyano substituierten $C_2$-$C_{12}$-Alkenylrest, einen $C_2$-$C_{12}$-Alkinylrest, einen durch Halogen oder Cyano substituierten $C_2$-$C_{12}$-Alkinylrest, einen $C_3$-$C_6$-Cycloalkylrest oder eine der Gruppen $-(CH_2)_mCO_2M$, $-(CH_2)_mCO_2R^1$ oder $-(CH_2)_mCONR^2R^3$ oder

$Y^5$ und $Y^6$ zusammen mit dem benachbarten Kohlenstoffatomen einen $C_3$-$C_6$-Cycloalkylrest,

W ein Sauerstoffatom oder die Gruppe $=N-R^2$,

Z einen der Thiadiazolylreste

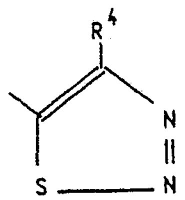

oder

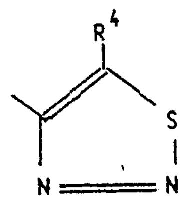

m 0, 1, 2, 3 oder 4,
M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

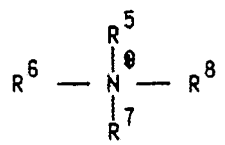

,

$R^1$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,
$R^2$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halo gen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,
$R^3$ ein Wasserstoffatom, einen $C_1$-$C_{18}$-Aklylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein-oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-Alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest oder
$R^2$ und $R^3$ gemeinsam mit dem benachbarten Stickstoffatom eine Morpholinogruppe, eine Piperidinogruppe oder eine Pyrrolidinogruppe,
$R^4$ ein Wasserstoffatom, ein Halogenatom, eine Cyanogruppe, einen $C_1$-$C_{18}$-Alkylrest, einen ein- oder mehrfach, gleich oder verschieden durch Halogen, Cyano oder eine der Gruppen $-OR^1$ oder $-S(O)_nR^1$ substituierten $C_1$-$C_{18}$-Alkylrest, einen $C_2$-$C_{12}$-Alkenylrest, einen ein- oder mehrfach, gleich oder verschieden durch Halogen, Cyano oder eine der Gruppen $-OR^1$ oder $-S(O)_nR^1$ substituierten $C_2$-$C_{12}$-Alkenylrest, einen $C_2$-$C_{12}$-Alkinylrest, einen ein- oder mehrfach, gleich oder verschieden durch Halogen, Cyano oder eine der Gruppen $-OR^1$ oder $-S(O)_nR^1$ substituierten $C_2$-$C_{12}$-Alkinylrest, einen Phenylrest, einen ein- oder mehrfach, gleich oder verschieden durch Halogen, Cyano, $C_1$-$C_4$-Alkyl oder eine der Gruppen $-OR^1$ oder $-S(O)_nR^1$

substituierten Phenylrest, einen Phenyl-$C_1$-$C_2$-alkylrest, einen-ein- oder mehrfach, gleich oder verschieden durch Halogen Cyano, $C_1$-$C_4$-Alkyl oder eine der Gruppen $-OR^1$ oder- $S(O)_nR^1$ substituierten Phenyl-$C_1$-$C_2$-alkylrest oder eine der Gruppen $-(CH_2)_mCO_2R^1$, $-COR^1$, $-(CH_2)_mCO_2M$, $-(CH_2)_mCONR^2R^3$, $-NHCONR^2R^3$, $-NHCO_2R^1$, $-NHCOR^1$, $-NHSO_2R^1$, $-(CH_2)_mNR^2R^3$, $-S(O)_nR^1$, $-OR^1$, $-CH=NNHR^1$ oder $-CH=NOR^1$,

$R^5$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest,

$R^6$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest,

$R^7$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest,

$R^8$ ein Wasserstoffatom, einen $C_1$-$C_8$-Alkylrest, einen ein- oder mehrfach, gleich oder verschieden durch Halogen, Hydroxy, Cyano oder $C_1$-$C_6$-Alkoxy substituierten $C_1$-$C_8$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituiertem Phenyl-$C_1$-$C_2$-alkylrest, und

n 0, 1 oder 2

bedeuten, und die, falls W für ein Sauerstoffatom steht, daraus abgeleiteten Salze der allgemeinen Formel II

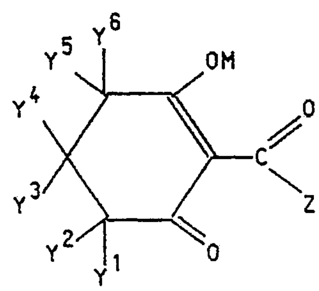

(II) ,

in der M, $Y^1$, $Y^2$, $Y^3$, $Y^4$, $Y^5$, $Y^6$ und Z die unter der allgemeinen Formel I genannten Bedeutungen haben.

2. Verfahren zur Herstellung von Verbindungen der allgemeinen Formel I, dadurch gekennzeichnet, daß man

A) falls W ein Sauerstoffatom bedeutet, eine Verbindung der allgemeinen Formel III

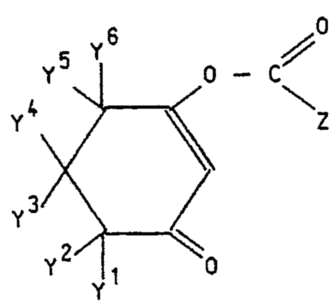

(III) ,

in der $Y^1$, $Y^2$, $Y^3$, $Y^4$, $Y^5$, $Y^6$ und Z die unter der allgemeinen Formel I genannten Bedeutungen haben, einer

katalytisch geförderten Umlagerungsreaktion unterwirft oder

B) falls W die Gruppe =N-$R^2$ bedeutet, eine Verbindung der allgemeinen Formel IV

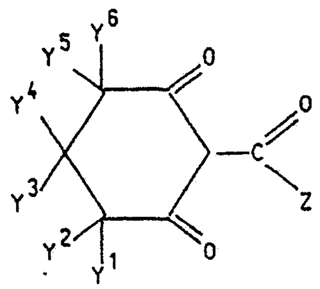

(IV) ,

in der $Y^1$, $Y^2$, $Y^3$, $Y^4$, $Y^5$, $Y^6$ und Z die unter der allgemeinen Formel I genannten Bedeutungen haben, mit Aminen der allgemeinen Formel V

$H_2N - R^2$ (V),

in der $R^2$ die unter der allgemeinen Formel I genannte Bedeutung hat, kondensiert.

3. Mittel mit herbizider und pflanzenwachstumsregulierender Wirkung, dadurch gekennzeichnet, daß sie mindestens eine Verbindung gemäß Anspruch 1 enthalten.

4. Verwendung von Mitteln gemäß Anspruch 3 zur Bekämpfung monokotyler und dikotyler Unkrautarten in landwirtschaftlichen Hauptkulturen.

5. Verwendung von Mitteln gemäß Anspruch 3 zur Wachstumsbeeinflussung von Kulturpflanzen.

6. Verfahren zur Herstellung von Mitteln gemäß Anspruch 3, dadurch gekennzeichnet, daß man eine oder mehrere Verbindungen gemäß Anspruch 1 mit Träger- und/oder sonstigen Hilfsstoffen vermischt.

Europäisches Patentamt

# EUROPÄISCHER RECHERCHENBERICHT

Nummer der Anmeldung

EP 89 10 6994

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 395 263 (SCHERING AG)<br>* Insgesamt *<br>--- | 1-6 | C 07 D 285/06<br>A 01 N 43/82 |
| D,Y | EP-A-0 137 963 (STAUFFER CHEMICAL)<br>* Insgesamt *<br>--- | 1-6 | |
| P,X | EP-A-0 283 261 (ICI)<br>* Insgesamt *<br>----- | 1-6 | |
| | | | **RECHERCHIERTE SACHGEBIETE (Int. Cl.4)** |
| | | | C 07 D 285/00 |

Der vorliegende Recherchenbericht wurde für alle Patentansprüche erstellt

| Recherchenort | Abschlußdatum der Recherche | Prüfer |
|---|---|---|
| DEN HAAG | 11-07-1989 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)